# EUROPÄISCHE PATENTSCHRIFT

(11) **EP 1 487 836 B1**
(45) Veröffentlichungstag und Bekanntmachung des Hinweises auf die Patenterteilung: **06.09.2006**
(21) Anmeldenummer: 03714788.1
(22) Anmeldetag: 07.03.2003
(51) Int. Cl.: C07D 471/04, C07D 497/04, A61K 31/55, A61P 9/10

(54) **ETHAN-1,2-DIAMINIUM-BIS((2R)-2-BROM-3-PHENYLPROPANOAT), VERFAHREN ZU DESSEN HERSTELLUNG UND DESSEN VERWENDUNG**
ETHANE-1,2-DIAMINO-BIS (2R)-2-BROMO-3-PHENYLPROPANOATE|, METHOD FOR PRODUCTION AND USE THEREOF
ETHAN-1,2-DIAMINIUM-BIS (2R)-2-BROM-3-PHENYLPROPIONATE|, SON PROCEDE DE PRODUCTION ET SON UTILISATION

(30) Priorität: 19.03.2002 DE 10212198
(43) Veröffentlichungstag der Anmeldung: 22.12.2004
(73) Patentinhaber: Sanofi-Aventis Deutschland GmbH, 65929 Frankfurt am Main (DE)
(72) Erfinder: MANERO, Javier, 65835 Liederbach (DE); METZENTHIN, Tobias, 65719 Hofheim (DE); GAULER, Rainer, 65835 Liederbach (DE)
(86) Internationale Anmeldenummer: PCT/EP2003/002353
(87) Internationale Veröffentlichungsnummer: WO 2003/078434

(56) Entgegenhaltungen:
- EP-A- 0 481 522
- WO-A-99/42438
- DATABASE CAPLUS [Online] CHEMICAL ABSTRACTS SERVICE, COLUMBUS, OHIO, US; Zugangsnummer 1990:99564, XP002244634 & JP 01 215805 A (TOHO TITANIUM CO., LTD.) 29. August 1989 (1989-08-29)

## Beschreibung

Chirale Verbindungen werden als Bausteine für die Synthese von pharmazeutischen Wirkstoffen eingesetzt. Für den Einsatz ist es erstrebenswert, daß diese Verbindungen lagerstabil sind und sich zudem einfach herstellen und reinigen lassen, um eine konstante Qualität sicherzustellen, häufige Kontrollen der Materialien zur Protokollierung der Produktqualität zu vermeiden, die Notwendigkeit von Kühllagern und/oder Kühltransporten zu vermeiden, ein leichtes Einfüllen in Produktionsanlagen und/oder die einfache Reinigung von benutzten Behältern zu gewährleisten.

Dementgegen lassen sich viskose Öle häufig nur schwer umfüllen oder genau abwiegen. Außerdem ist die Reinigung von Ölen oder viskosen Flüssigkeiten in vielen Fällen nur mit erheblichem apparativen Aufwand durchführbar. Zusätzlich können Öle ein nicht optimales Verhalten beim Auflösen in Lösungsmitteln zeigen, weshalb die Durchmischung von Flüssigkeiten mit unterschiedlichen Dichten und Viskositäten mit besonderer Sorgfalt überwacht werden muß.

(2R)-2-Brom-3-phenylpropionsäure ist ein zähes Öl, das diverse der oben aufgezählten nachteiligen Eigenschaften aufweist. Ein Einsatz von (2R)-2-Brom-3-phenylpropionsäure im technischen Maßstab ist daher mit Schwierigkeiten und zusätzlichen Kosten verbunden.

Aufgabe der vorliegenden Erfindung ist es, (2R)-2-Brom-3-phenylpropionsäure in einer Form bereitzustellen, die die oben genannten Nachteile nicht aufweist und die in technischen Synthesen eingesetzt werden kann.

Die Aufgabe wird erfindungsgemäß gelöst durch die Bereitstellung des Salzes (Ethan-1,2-diaminium-bis[(2R)-2-brom-3-phenylpropanoat]) in reiner Form und unter Erhalt der Enantiomerenreinheit.
Überraschenderweise wurde gefunden, dass sich aus (2R)-2-Brom-3-phenylpropionsäure und Ethylendiamin in 2-Propanol ein Salz (Ethan-1,2-diaminium-bis[(2R)-2-brom-3-phenylpropanoat]) in der Stöchiometrie 2:1 in guter Ausbeute und ausgezeichneter chemischer sowie enantiomerer Reinheit isolieren lässt.

Gegenstand der vorliegenden Anmeldung ist demgemäß Ethan-1,2-diaminium-bis[(2R)-2-brom-3-phenylpropanoat].

Gegenstand der Erfindung ist ferner ein Verfahren zur Herstellung von Ethan-1,2-diaminium-bis[(2R)-2-brom-3-phenylpropanoat], dadurch gekennzeichnet, dass
a) (2R)-2-Brom-3-phenylpropionsäure, Ethylendiamin und 2-Propanol vermischt werden, wobei Ethan-1,2-diaminium-bis[(2R)-2-brom-3-phenylpropanoat] auskristallisiert, und
b) das auskristallisierte Ethan-1,2-diaminium-bis[(2R)-2-brom-3-phenylpropanoat] isoliert wird.

Vorzugsweise besitzt das in dem erfindungsgemäßen Verfahren hergestellte Ethan-1,2-diaminium-bis[(2R)-2-brom-3-phenylpropanoat] eine höhere Reinheit als das Ausgangsmaterial (2R)-2-Brom-3-phenylpropionsäure.

Vorzugsweise verläuft das erfindungsgemäße Verfahren unter Erhalt oder Steigerung der Enantiomerenreinheit.

In Schritt b) kann das auskristallisierte Ethan-1,2-diaminium-bis[(2R)-2-brom-3-phenylpropanoat] isoliert werden, indem es abfiltriert oder abgesaugt wird, optional kann das abgesaugte oder abfiltrierte Produkt anschliessend mit 2-Propanol gewaschen und getrocknet werden.

Das Ausgangsmaterial (2R)-2-Brom-3-phenylpropionsäure ist beispielsweise von der Firma Zambon Group spa (I-20091 Bresso, Italien) oder der Firma Kaneka (Osaka, Japan) erhältlich.
Ethan-1,2-diaminium-bis[(2R)-2-brom-3-phenylpropanoat] kann beispielsweise für die Synthese von ACE/NEP-Inhibitoren verwendet werden, beispielsweise zu einer Verbindung der Formel (I) umgesetzt, 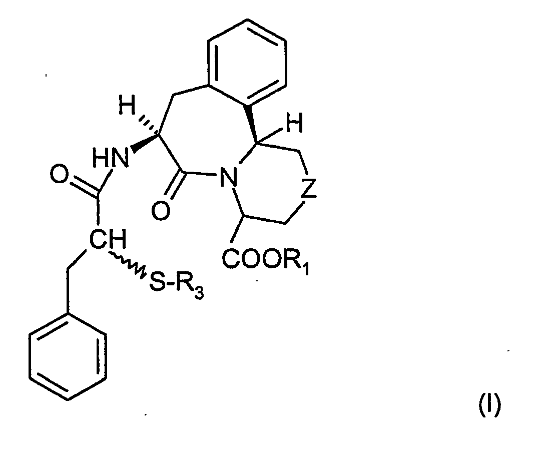 wobei
R₁ H, -CH₂O-C(O)C(CH₃)₃, C₁-C₄-Alkyl, oder eine Aryl-Y-Gruppe ist, wobei Y eine Einfachbindung oder C₁-C₄-Alkyl ist,
R₃H, Acetyl, -CH₂O-C(O)C(CH₃)₃ oder Benzoyl ist, und
Z (CH₂)ₙ, -O-, S, NR₆ oder N-C(O)R₇ , wobei n eine ganze Zahl 0 oder 1 ist, R₆ H, C₁₋C₄-Alkyl oder eine Aryl-Y-Gruppe ist, und R₇CF₃, C₁-C₁₀-Alkyl oder eine Aryl-Y-Gruppe ist,
wobei vorzugsweise R₁ H, R₃ H oder Acetyl, und Z (CH₂)ₙ, und n 0 ist,
und wobei die CH-SR₃-Gruppe die (R)- oder die (S)-Konfiguration haben kann, und vorzugsweise die (S)-Konfiguration hat,
und wobei die Verbindung der Formel (I) vorzugsweise durch eine Verbindung der Formel (la) 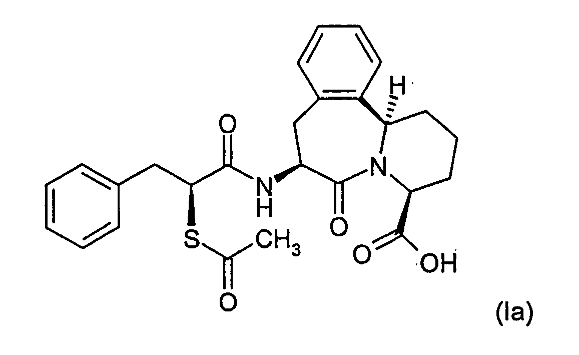 oder der Formel (Ib) 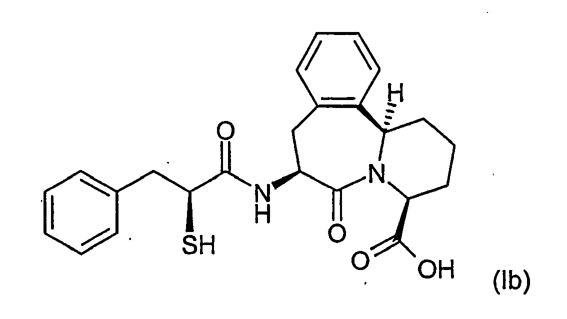 beschrieben wird,
dadurch gekennzeichnet, daß
entweder zunächst aus Ethan-1,2-diaminium-bis[(2R)-2-brom-3-phenylpropanoat] intermediär oder in einem separaten Schritt (2R)-2-Brom-3-phenylpropionsäure unter Zusatz einer Säure nach ansich bekannten Methoden freigesetzt wird, und anschliessend (2R)-2-Brom-3-phenylpropionsäure mit einer Verbindung der Formel (II) 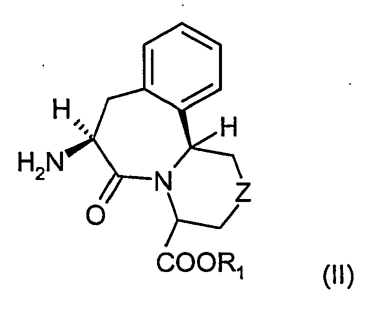 umgesetzt und mit einem Thioacetat zu einer Verbindung der Formel (I) reagiert, und das Thioacetat optional zum freien Thiol umgesetzt wird,
oder dadurch gekennzeichnet, daß (2R)-2-Brom-3-phenylpropionsäure wie beispielsweise in der europäischen Patentanmeldung EP 1056715 zu (S)-2-(Acetylthio)-3-phenylpropionsäure umgesetzt wird und anschliessend wie beispielsweise im europäischen Patent EP 481522 beschrieben mit einer Verbindung der Formel (II) zu einer Verbindung der Formel (I) umgesetzt wird, und das Thioacetat optional zum freien Thiol umgesetzt wird.
"C₁-C₄-Alkyl" bedeutet eine gesättigte geradkettige oder verzweigte Kohlenwasserstoffkette aus 1-4 Kohlenstoffatomen und beinhaltet beispielsweise Methyl, Ethyl, Propyl, Isopropyl, n-Butyl, Isobutyl, tert-Butyl. Der Ausdruck "C₁-C₁₀₋Alkyl" bedeutet einen gesättigten geradkettigen oder verzweigten Kohlenwasserstoffrest mit 1-10 Kohlenstoffatomen, beispielsweise Methyl, Ethyl, Propyl, Isopropyl, n-Butyl, Isobutyl, tert-Butyl, Pentyl, Isopentyl, Hexyl, 2,3-Dimethyl-2-butyl, Heptyl, 2,2-Dimethyl-3-pentyl, 2-Methyl-2-hexyl, Octyl, 4-Methyl-3-heptyl.
"Aryl-Y-" bedeutet einen Arylrest, der mit einer Gruppe Y substituiert ist, wobei Y eine direkte Bindung oder eine C₁-C₄-Alkylgruppe sein kann. Aryl bedeutet eine Phenyl- or Naphthyl-Gruppe, die unsubstituiert oder substituiert ist mit 1-3 Substituenten aus der Gruppe Methylenedioxy, Hydroxy, C₁-C₄-Alkoxy, Fluor und Chlor. Beispielsweise bedeutet der Ausdruck "Aryl-Y-" Phenyl, Naphthyl, Phenylmethyl, Benzyl, Phenylethyl, p-Methoxybenzyl, p-Fluorobenzyl oder p-Chlorobenzyl. "Aryl-.Y-" kann auch eine Diphenylmethyl-Gruppe bedeuten.

Ethan-1,2-diaminium-bis[(2R)-2-brom-3-phenylpropanoat] kann ferner zur Herstellung der Verbindung Omapatrilat ([4S-[4α(R*), 7α, 10aβ]]-Octahydro-4-[(2-mercapto-1-oxo-3-phenylpropyl)amino]-5-oxo-7H-pyrido[2,1-b][1,3]-thiazepine-7-carboxylic acid) der Formel (III) 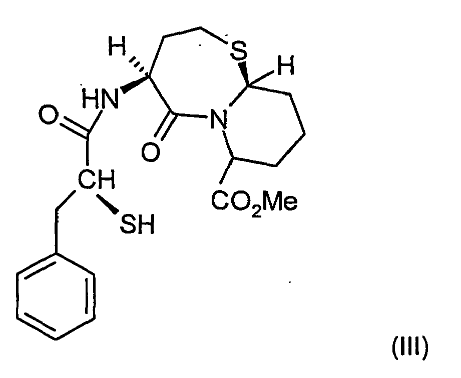 benutzt werden, wobei zunächst aus Ethan-1,2-diaminium-bis[(2R)-2-brom-3-phenylpropanoat] intermediär oder in einem separaten Schritt (2R)-2-Brom-3-phenyl-propionsäure unter Zusatz einer Säure nach ansich bekannten Methoden freigesetzt wird, und anschliessend (2R)-2-Brom-3-phenylpropionsäure zu (S)-2-(Acetylthio)-3-phenylpropionsäure umgesetzt wird und entsprechend J. Med. Chem., 1999 (42), 305-311, oder J. Med. Chem., 1997 (40) 1570-1577 in einem weiteren Schritt (Acetylthio)-3-phenylpropionsäure mit einer Verbindung der Formel (IV) 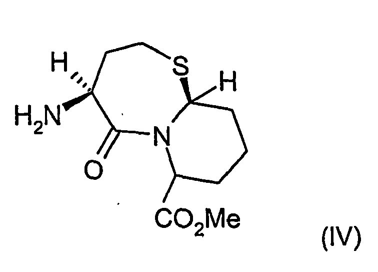 zur Verbindung der Formel (III) umgesetzt wird.

Die Säure ist eine organische oder anorganische Säure, vorzugsweise eine anorganische Säure, beispielsweise eine Mineralsäure wie H₂SO₄ oder HCl.

Es wurde gefunden, daß bei Verwendung von 2-Propanol als Lösungsmittel und Ethylendiamin als Amin die chemische Reinheit des Produktes gegenüber dem Ausgangsmaterial von 92,5% auf 98% gesteigert, eine gute Ausbeute von 58% und eine sehr gute Enantiomerenreinheit von 98,6% ee (ausgehend von einer Enantiomerenreinheit des Ausgangsmaterials von 83,4% ee) erzielt werden kann.

Gegenstand der vorliegenden Anmeldung ist demgemäß Ethan-1,2-diaminium-bis[(2R)-2-brom-3-phenylpropanoat] einer chemischen Reinheit größer als 92,5%, vorzugsweise größer oder gleich 98%.

Gegenstand der vorliegenden Anmeldung ist ausserdem Ethan-1,2-diaminium-bis[(2R)-2-brom-3-phenylpropanoat] einer Enantiomerenreinheit größer als 83,4% ee, vorzugsweise größer oder gleich 95% ee, besonders bevorzugt größer oder gleich 98% ee.

Bei der Zugabe von basischen Agenzien wie beispielsweise Aminen kann es zu einer Eliminierung von Bromwasserstoff aus (2R)-2-Brom-3-phenylpropionsäure kommen, wobei Zimtsäure entsteht und die Enantiomerenreinheit des Produktes verringert wird. Eine solche Eliminierung wird bei der Verwendung von Ethylendiamin als Amin in 2-Propanol als Lösungsmittel nicht beobachtet. Das Produkt Ethan-1,2-diaminium-bis[(2R)-2-brom-3-phenylpropanoat] wird daher in ausgezeichneter Enantiomerenreinheit erhalten.

Darüberhinaus ist bei der Wahl von Lösungsmittel und Amin zu beachten, daß das Produktsalz aus dem Reaktionsgemisch auskristallisiert, um erstens leicht isolierbar zu sein und wobei zweitens das Reaktionsgleichgewicht auf die Seite des Produktes verschoben wird.

Bei Verwendung anderer Lösungsmittel und/oder Aminen wird entweder die Reinheit des Produktes gegenüber dem Ausgangsmaterial nicht gesteigert, oder die Reaktion läuft nicht ab, oder die Ausbeute ist gering, oder es findet als Nebenreaktion eine Eliminierung statt.
Die folgenden Beispiele sollen die Erfindung näher erläutern, ohne sie zu auf diese Ausführungsformen zu beschränken.

### Bespiel 1: Synthese von Ethan-1,2-diaminium-bis[(2R)-2-brom-3-phenylpropanoat] in 2-Propanol

5 g (2R)-2-Brom-3-phenylpropionsäure (HPLC: 92,5%) wurden in 20 ml 2-Propanol gelöst und bei Raumtemperatur mit 0,87 g Ethylendiamin versetzt. Nach ca. 150 Minuten Nachrührzeit wurde das ausgefallene Produkt abgesaugt und mit 2-Propanol gewaschen. Das Produkt wurde im Vakuum bei 45°C über Nacht getrocknet. Die Reinheit des isolierten Produktes betrug laut HPLC 98%, der Gehalt am R-Enantiomeren 98,6%, und die Ausbeute 58%

### Beispiel 2: Charakterisierung von Ethan-1,2-diaminium-bis[(2R)-2-brom-3-phenylpropanoat]

Die Identität von Ethan-1,2-diaminium-bis[(2R)-2-brom-3-phenylpropanoat] wurde durch HPLC-Vergleich mit einem Muster des Ausgangsmaterials festgestellt. Hierbei wurde die Qualität der Säurekomponente des Salzes überprüft, es war keine Zimtsäure oder andere neue, unbekannte Substanzen zu detektieren. Die 1:2-Stöchiometrie wurde über eine Tritration mit 0,1 N NaOH in Wasser als Lösungsmittel verifiziert: Es waren zwei pH-Stufen zu beobachten, die beide den selben Verbrauch an NaOH aufweisen. Der Drehwert von Ethan-1,2-diaminium-bis[(2R)-2-brom-3-phenylpropanoat] betrug [α]²⁰_{D}= +5,6° (c=2 in DMF), der Schmelzpunkt betrug 147°C.

**Tabelle 1: ¹H-NMR-Daten der Verbindung Ethan-1,2-diaminium bis[(2R)-2-brom-3-phenylpropanoat] (chemische Verschiebungen)**

| | | |
|---|---|---|
| Position innerhalb von | ¹³C δ (ppm) | ¹H δ (ppm) |
| [Phenyl-CH₂-CHBr-COO-] | | |
| [⁺H₃N-CH₂-CH₂-NH₃⁺] | | |
| -COO- | 176,24 | - |
| -CHBr- | 52,52 | 4,39 t |
| -CH₂- | 41,03 | 3,30 dd |
| | | 3,15 dd |
| Phenyl- | 137,75 | 7,26 d |
| | 128,73 | 7,32 t |
| | 128,18 | 7,26 t |
| | 126,59 | |
| ⁺H₃N-CH₂-CH₂-NH₃⁺ | 36,04 | 3,28 s |
| Multiplizitäten: | | |
| s = Singulett, d=Dublett, t=Triplett, dd=Doppeldublett | | |

### Beispiel 3: Syntheseversuch von Ethan-1,2-diaminium-bis[(2R)-2-brom-3-phenylpropanoat] in Ethylacetat

5 g (2R)-2-Brom-3-phenylpropionsäure (HPLC: 92,5%) wurden in 20 ml Ethylacetat gelöst und bei Raumtemperatur mit 0,87 g Ethylendiamin. Nach ca. 150 Minuten Nachrührzeit wurde keine Fällung des Produktes beobachtet. Eine HPLC-Analyse der Lösung zeigte, daß (2R)-2-Brom-3-phenylpropionsäure unverändert vorlag.

### Beispiel 4: Syntheseversuch von Cyclohexylamin-[(2R)-2-brom-3-phenylpropanoat] in 2-Propanol

2,38 g (2R)-2-Brom-3-phenylpropionsäure (HPLC: 92,5%) wurden in 15 ml 2-Propanol gelöst und bei Raumtemperatur mit 0,65 g Cyclohexylamin versetzt. Nach ca. 150 Minuten Nachrührzeit wurde das ausgefallene Produkt abgesaugt, mit 2-Propanol gewaschen und im Vakuum bei 45°C über Nacht getrocknet. Die Reinheit des isolierten Produktes betrugt laut HPLC 92%. Unter den Verunreinigungen fand sich auch Zimtsäure. Die Ausbeute lag lediglich bei 22%.

### Beispiel 5: Syntheseversuch von Triethylammonium-[(2R)-2-brom-3-phenylpropanoat] in Ethylacetat

5 g rohe (2R)-2-Brom-3-phenylpropionsäure (HPLC: 92,5%) wurden in 20 ml Ethylacetat gelöst und bei Raumtemperatur mit 2,7 ml Triethylamin versetzt. Nach ca. 150 Minuten Nachrührzeit war keine Fällung des Produktes zu beobachten. Eine HPLC-Analyse der Lösung zeigte, daß die (2R)-2-Brom-3-phenylpropionsäure vollständig zu Zimtsäure reagiert hatte.

### Beispiel 6: Synthese von Dicyclohexylammonium-[(2R)-2-brom-3-phenylpropanoat] in 2-Propanol

5 g (2R)-2-Brom-3-phenylpropionsäure (HPLC: 92,5%) wurden in 20 ml 2-Propanol gelöst und bei Raumtemperatur mit 4,25 g Dicyclohexylamin versetzt. Nach ca. 150 Minuten Nachrührzeit wurde das ausgefallene Salz abgesaugt und mit 2-Propanol gewaschen. Das Produkt wurde im Vakuum bei 45°C über Nacht getrocknet. Die Reinheit des isolierten Produktes betrug laut HPLC 94%, die Ausbeute betrug 44%

### Beispiel 7: Synthese von Dicyclohexylammonium-[(2R)-2-brom-3-phenylpropanoat] in Ethylacetat

5 g (2R)-2-Brom-3-phenylpropionsäure (HPLC: 92,5%) wurden in 20 ml Ethylacetat bei Raumtemperatur mit 4,25 g Dicyclohexylamin versetzt. Nach ca. 150 Minuten Nachrührzeit wurde das ausgefallene Salz abgesaugt und mit Ethylacetat gewaschen. Das Produkt wurde im Vakuum bei 45°C über Nacht getrocknet. Die Reinheit des isolierten Produktes betrug laut HPLC nur 86%. Unter den Verunreinigungen fand sich auch Zimtsäure. Die Ausbeute betrug 32%.

### Beispiel 8: Synthese von Dicyclohexylammonium-[(2R)-2-brom-3-phenylpropanoat] in Toluol

5 g (2R)-2-Brom-3-phenylpropionsäure (HPLC: 92,5%) wurden in 20 ml Toluol bei Raumtemperatur mit 4,25 g Dicyclohexylamin versetzt. Nach ca. 150 Minuten Nachrührzeit wurde das ausgefallene Salz abgesaugt und mit Toluol gewaschen. Das Produkt wurde im Vakuum bei 45°C über Nacht getrocknet. Die Reinheit des isolierten Produktes betrug laut HPLC nur 81 %. Unter den Verunreinigungen fand sich auch Zimtsäure. Die Ausbeute betrug 31%.

### Beispiel 9: Syntheseversuch von Triethylammonium-[(2R)-2-brom-3-phenylpropanoat] in Toluol

5 g (2R)-2-Brom-3-phenylpropionsäure (HPLC: 92,5%) wurden in 20 ml Toluol bei Raumtemperatur mit 2,7 ml Triethylamin versetzt. Nach ca. 150 Minuten Nachrührzeit war keine Fällung des Produktes zu beobachten. Eine HPLC-Analyse der Lösung zeigte, daß die (2R)-2-Brom-3-phenylpropionsäure vollständig zu Zimtsäure reagiert hatte.

### Beispiel 10: Synthese von Dicyclohexylammonium-[(2R)-2-brom-3-phenylpropanoat] in Diisopropylether

5 g (2R)-2-Brom-3-phenylpropionsäure (HPLC: 92,5%) wurden in 20 ml Diisopropylether bei Raumtemperatur mit 4,25 g Dicyclohexylamin versetzt. Nach ca. 150 Minuten Nachrührzeit wurde das ausgefallene Salz abgesaugt und mit Diisopropylether gewaschen. Das Produkt wurde im Vakuum bei 45°C über Nacht getrocknet. Die Reinheit des isolierten Produktes betrug laut HPLC nur 85%. Unter den Verunreinigungen fand sich auch Zimtsäure. Die Ausbeute betrug 55%.

### Beispiel 11: Synthese von Dicyclohexylammonium-[(2R)-2-brom-3-phenylpropanoat] in Ethanol

5 g (2R)-2-Brom-3-phenylpropionsäure (HPLC: 92,5%) wurden in 20 ml Ethanol bei Raumtemperatur mit 4,25 g Dicyclohexylamin versetzt. Nach ca. 150 Minuten Nachrührzeit wurde das ausgefallene Salz abgesaugt und mit Ethanol gewaschen. Das Produkt wurde im Vakuum bei 45°C über Nacht getrocknet. Die Reinheit des isolierten Produktes betrug laut HPLC 96%. Unter den Verunreinigungen fand sich auch Zimtsäure. Die Ausbeute lag lediglich bei 12%.

### Beispiel 12: Syntheseversuch von N,N,N',N'-Tetramethylethan-1,2-diaminium-bis[(2R)-2-brom-3-phenylpropanoat] in 2-Propanol

5 g (2R)-2-Brom-3-phenylpropionsäure (HPLC: 92,5%) wurden in 20 ml 2-Propanol gelöst und bei Raumtemperatur mit 1,39 g Tetramethylethylendiamin versetzt. Nach ca. 150 Minuten Nachrührzeit war keine Fällung des Produktes zu beobachten. Eine HPLC-Analyse der Lösung zeigte, daß (2R)-2-Brom-3-phenylpropionsäure unverändert vorlag.

### Beispiel 13: Syntheseversuch von N,N,N',N'-Tetramethylethan-1,2-diaminium-bis[(2R)-2-brom-3-phenylpropanoat] in Toluol

5 g (2R)-2-Brom-3-phenylpropionsäure (HPLC: 92,5%) wurden in 20 ml Toluol gelöst und bei Raumtemperatur mit 1,39 g Tetramethylethylendiamin versetzt. Nach ca. 150 Minuten Nachrührzeit war keine Fällung des Produktes zu beobachten. Eine HPLC-Analyse der Lösung zeigte, daß die (2R)-2-Brom-3-phenylpropionsäure vollständig zu Zimtsäure reagiert hatte.

### Beispiel 14: Herstellung von (R)-2-Brom-3-phenylpropionsäure aus Ethan-1,2-diaminium-bis[(2R)-2-brom-3-phenylpropanoat]

In einem 500 ml Vierhalskolben mit Ablaßventil wurden 95,0 g Ethan-1,2-diaminium-bis[(2R)-2-brom-3-phenylpropanoat] (0,0675mol) vorgelegt und mit 105 ml Diisopropyletheter und 105 ml Wasser versetzt. Die entstehende weiße Suspension wurde mit 15 ml 30%ige Salzsäure von pH 6,7 auf pH 1,5 eingestellt, wobei sich die Suspension auflöste. Anschliessend wurde das Reaktionsgemisch 30 Minuten bei pH 1,5 und Raumtemperatur (ca. 20°C) gerührt, und die Phasen getrennt. Die organische Phase wurde einmal mit
35 ml Wasser extrahiert, die wäßrige Phase verworfen. Aus der organischen Phase wurde das Lösungsmittel Diisopropylether und Wasser-Reste destillativ entfernt. Der Rückstand wurde mit 90 ml Aceton versetzt und erneut destilliert, wobei Aceton und Diisopropylether-Reste abdestillierten. Die Ausbeute war quantitativ bezüglich (R)-2-Brom-3-phenylpropionsäure.

## Patentansprüche

1. Ethan-1,2-diaminium-bis[(2R)-2-brom-3-phenylpropanoat].

2. Ethan-1,2-diaminium-bis[(2R)-2-brom-3-phenylpropanoat] gemäß Anspruch 1 in einer chemischen Reinheit von größer oder gleich 98%.

3. Ethan-1,2-diaminium-bis[(2R)-2-brom-3-phenylpropanoat] gemäß einem der Ansprüche 1 oder 2 in einer Enantiomerenreinheit größer als 83,4% ee.

4. Ethan-1,2-diaminium-bis[(2R)-2-brom-3-phenylpropanoat] gemäß Anspruch 3 in einer Enantiomerenreinheit größer oder gleich 95% ee, vorzugsweise größer oder gleich 98% ee.

5. Verfahren zur Herstellung von Ethan-1,2-diaminium-bis[(2R)-2-brom-3-phenylpropanoat], **dadurch gekennzeichnet, daß**
a) (2R)-2-Brom-3-phenylpropionsäure, Etylendiamin und 2-Propanol vermischt werden, wobei Ethan-1,2-diaminium-bis[(2R)-2-brom-3-phenylpropanoat] auskristallisiert, und
b) das auskristallisierte Ethan-1,2-diaminium-bis[(2R)-2-brom-3-phenylpropanoat] isoliert wird.

6. Verfahren gemäß Anspruch 5, wobei das Ethan-1,2-diaminium-bis[(2R)-2-brom-3-phenylpropanoat]-Produkt eine höhere chemische Reinheit als das Ausgangsmaterial (2R)-2-Brom-3-phenylpropionsäure hat.

7. Verfahren gemäß Anspruch 6, wobei die chemische Reinheit des Ethan-1,2-diaminium-bis[(2R)-2-brom-3-phenylpropanoat]-Produktes größer ist als 92,5 %.

8. Verfahren gemäß einem der Ansprüche 6 bis 7, wobei die chemische Reinheit des Ethan-1,2-diaminium-bis[(2R)-2-brom-3-phenylpropanoat]-Produktes mindestens 98 % beträgt.

9. Verfahren gemäß einem der Ansprüche 5 bis 8, wobei das auskristallisierte Ethan-1,2-diaminium-bis[(2R)-2-brom-3-phenylpropanoat] in Verfahrensschritt b) isoliert wird, indem es abfiltriert oder abgesaugt, mit 2-Propanol gewaschen und getrocknet werden.

10. Verwendung von Ethan-1,2-diaminium-bis[(2R)-2-brom-3-phenylpropanoat] gemäß einem der Ansprüche 1 bis 4 zur Herstellung von ACE/NEP-Inhibitoren.

11. Verfahren zur Herstellung einer Verbindung der Formel (I) 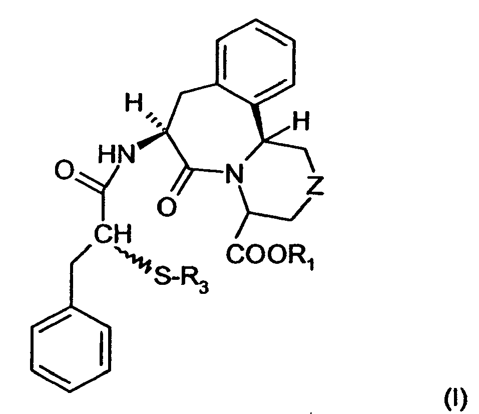 wobei
R₁ H, -CH₂O-C(O)C(CH₃)₃, C₁-C₄-Alkyl oder eine Aryl-Y-Gruppe ist, wobei Y eine Einfachbindung oder C₁-C₄-Alkyl ist,
R₃ H, Acetyl, -CH₂O-C(O)C(CH₃)₃ oder Benzoyl ist, und
Z (CH₂)ₙ, -O-, S, NR₆ oder N-C(O)R₇, wobei n eine ganze Zahl 0 oder 1 ist, R₆ H, C₁-C₄-Alkyl oder eine Aryl-Y-Gruppe ist, und R₇ CF₃, C₁-C₁₀-Alkyl oder eine Aryl-Y-Gruppe ist, wobei Aryl eine Phenyl- or Naphthyl-Gruppe ist, die unsubstituiert oder substituiert ist mit 1-3 Substituenten aus_der Gruppe Methylenedioxy, Hydroxy, C₁-C₄-Alkoxy, Fluor und Chlor,
**dadurch gekennzeichnet, daß**
entweder zunächst aus Ethan-1,2-diaminium-bis[(2R)-2-brom-3-phenylpropanoat] intermediär oder in einem separaten Schritt unter Zusatz einer Säure (2R)-2-Brom-3-phenylpropionsäure freigesetzt wird, anschliessend (2R)-2-Brom-3-phenylpropionsäure mit einer Verbindung der Formel (II) 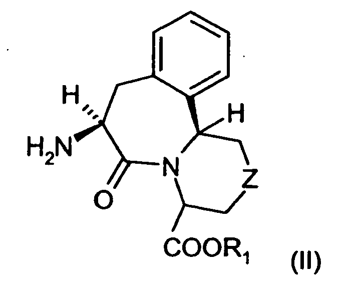 umgesetzt und mit einem Thioacetat zu einer Verbindung der Formel (I) reagiert, und das Thioacetat optional zum freien Thiol umgesetzt wird,
oder **dadurch gekennzeichnet, daß** (2R)-2-Brom-3-phenylpropionsäure zu (S)-2-(Acetylthio)-3-phenylpropionsäure umgesetzt wird, anschliessend mit einer Verbindung der Formel (II) zu einer Verbindung der Formel (I) umgesetzt wird, und das Thioacetat optional zum freien Thiol umgesetzt wird.

12. Verfahren gemäß Anspruch 11, wobei die Verbindung der Formel (I) durch eine Verbindung der Formel (la) 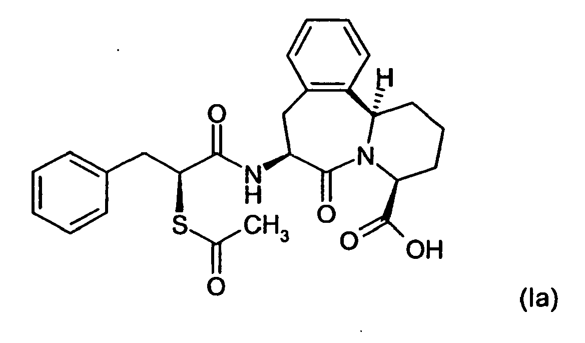 oder der Formel (Ib) 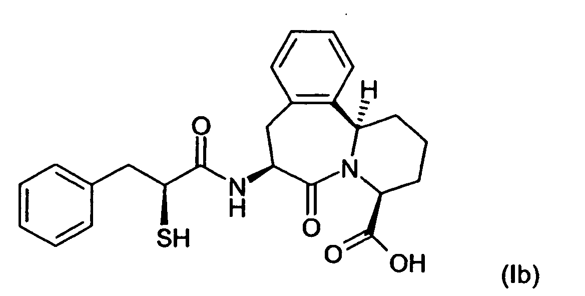 beschrieben wird.

13. Verfahren zur Herstellung der Verbindung der Formel (III) 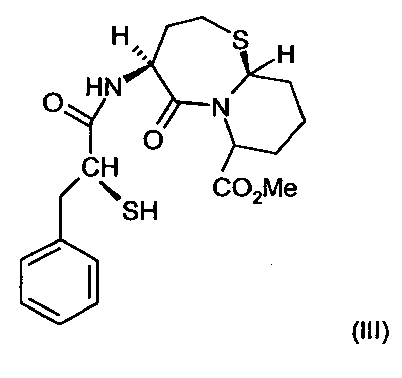 **dadurch gekennzeichnet, daß** zunächst aus Ethan-1,2-diaminium-bis[(2R)-2-brom-3-phenylpropanoat] intermediär oder in einem separaten Schritt unter Zusatz einer Säure (2R)-2-Brom-3-phenylpropionsäure freigesetzt wird, anschliessend (2R)-2-Brom-3-phönylpropionsäure zu (S)-2-(Acetylthio)-3-phenylpropionsäure umgesetzt wird, und in einem weiteren Schritt (Acetylthio)-3-phenylpropionsäure mit einer Verbindung der Formel (IV) 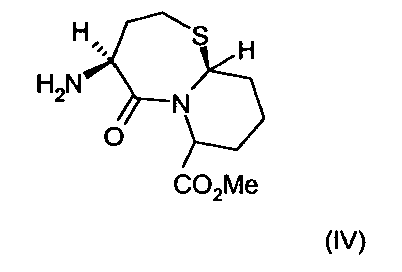 zur Verbindung der Formel (III) umgesetzt wird.

## Claims

1. Ethane-1,2-diaminium bis[(2R)-2-bromo-3-phenylpropanoate].

2. Ethane-1,2-diaminium bis[(2R)-2-bromo-3-phenylpropanoate] as claimed in claim 1 in a chemical purity of greater than or equal to 98%.

3. Ethane-1,2-diaminium bis[(2R)-2-bromo-3-phenylpropanoate] as claimed in one of claims 1 or 2 in an enantiomeric purity of greater than 83.4% ee.

4. Ethane-1,2-diaminium bis[(2R)-2-bromo-3-phenylpropanoate] as claimed in claim 3 in an enantiomeric purity of greater than or equal to 95% ee, preferably greater than or equal to 98% ee.

5. A process for the preparation of ethane-1,2-diaminium bis[(2R)-2-bromo-3-phenylpropanoate], which comprises
a) mixing (2R)-2-bromo-3-phenylpropionic acid, ethylenediamine and 2-propanol, ethane-1,2-diaminium bis[(2R)-2-bromo-3-phenylpropanoate] crystallizing, and
b) isolating the crystallized ethane-1,2-diaminium bis[(2R)-2-bromo-3-phenyl-propanoate].

6. The process as claimed in claim 5, the ethane-1,2-diaminium bis[(2R)-2-bromo- 3-phenylpropanoate] product having a higher chemical purity than the starting material (2R)-2-bromo-3-phenylpropionic acid.

7. The process as claimed in claim 6, the chemical purity of the ethane-1,2-diaminium bis[(2R)-2-bromo-3-phenylpropanoate] product being greater than 92.5 %.

8. The process as claimed in one of claims 6 to 7, the chemical purity of the ethane-1,2-di-aminium bis[(2R)-2-bromo-3-phenylpropanoate] product being at least 98 %.

9. The process as claimed in one of claims 5 to 8, the crystallized ethane-1,2-di-aminium bis[(2R)-2-bromo-3-phenylpropanoate] being isolated in process step b) by filtering off or filtering off with suction, washing with 2-propanol and drying.

10. The use of ethane-1,2-diaminium bis[(2R)-2-bromo-3-phenylpropanoate] as claimed in one of claims 1 to 4 for the preparation of ACE/NEP inhibitors.

11. A process for the preparation of a compound of the formula (I) 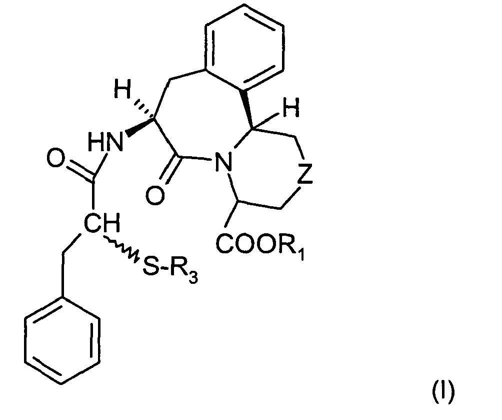 where
R₁ is H, -CH₂O-C(O)C(CH₃)₃, C₁-C₄-alkyl or an aryl-Y- group, where Y is a single bond or C₁-C₄-alkyl,
R₃ is H, acetyl, -CH₂O-C(O)C(CH₃)₃ or benzoyl, and
Z is (CH₂)ₙ, -O-, S, NR₆ or N-C(O)R₇, where n is an integer 0 or 1, R₆ is H, C₁-C₄-alkyl or an aryl-Y- group, and R₇ is CF₃, C₁-C₁₀-alkyl or an aryl-Y-group, where aryl is a phenyl or naphthyl group which is unsubstituted or substituted by 1-3 substituents from the group consisting of methylenedioxy, hydroxyl, C1-C4-alkoxy, fluorine and chlorine,
which comprises
either firstly releasing (2R)-2-bromo-3-phenylpropionic acid from ethane-1,2-di-aminium bis[(2R)-2-bromo-3-phenylpropanoate] intermediately or in a separate step with addition of an acid, then reacting (2R)-2-bromo-3-phenylpropionic acid with a compound of the formula (II) 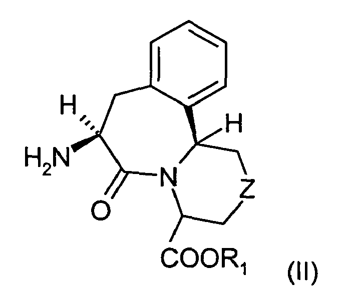 and reacting with a thioacetate to give a compound of the formula (I), and optionally reacting the thioacetate to give the free thiol,
or which comprises reacting (2R)-2-bromo-3-phenylpropionic acid to give (S)-2-(acetylthio)-3-phenylpropionic acid, then reacting with a compound of the formula (II) to give a compound of the formula (I), and optionally reacting the thioacetate to give the free thiol.

12. The process as claimed in claim 11, where the compound of the formula (I) is described by a compound of the formula (Ia) 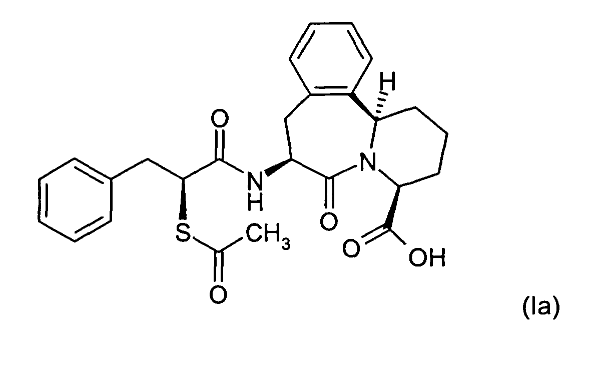 or of the formula (Ib)

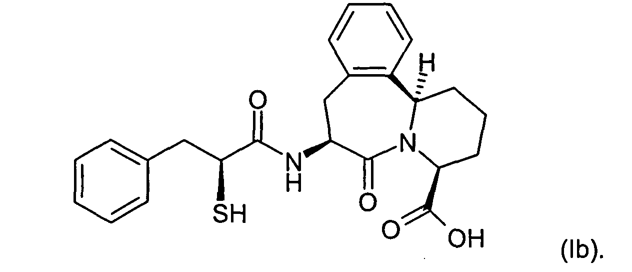

13. A process for the preparation of the compound of the formula (III) 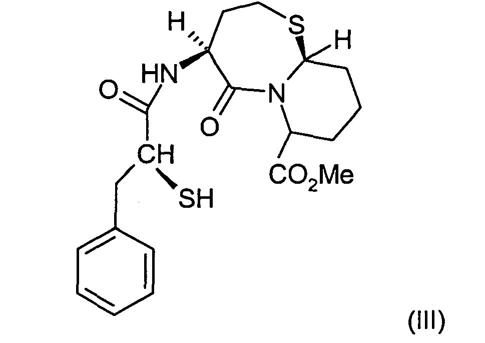 which comprises firstly releasing (2R)-2-bromo-3-phenylpropionic acid from ethane-1,2-diaminium bis[(2R)-2-bromo-3-phenylpropanoate] intermediately or in a separate step with addition of an acid, then reacting (2R)-2-bromo-3-phenylpropionic acid to give (S)-2-(acetylthio)-3-phenylpropionic acid, and in a further step reacting (acetylthio)-3-phenylpropionic acid with a compound of the formula (IV) 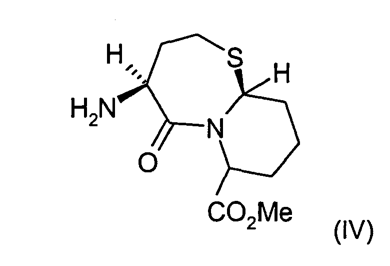 to give the compound of the formula (III).

## Revendications

1. Ethane-1,2-diaminium-bis[(2R)-2-bromo-3-phénylpropanoate].

2. Ethane-1,2-diaminium-bis[(2R)-2-bromo-3-phénylpropanoate] selon la revendication 1 en une pureté chimique supérieure ou égale à 98 %.

3. Ethane-1,2-diaminium-bis[(2R)-2-bromo-3-phénylpropanoate] selon l'une quelconque des revendications 1 ou 2 en une pureté énantiomérique supérieure à 83,4 % ee.

4. Ethane-1,2-diaminium-bis[(2R)-2-bromo-3-phénylpropanoate] selon la revendication 3 en une pureté énantiomérique supérieure ou égale à 95 % ee, de préférence supérieure ou égale à 98 % ee.

5. Procédé de préparation de l'éthane-1,2-diaminium-bis[(2R)-2-bromo-3-phénylpropanoate], **caractérisé en ce que**
a) de l'acide (2R)-2-bromo-3-phénylpropionique, de l'éthylènediamine et 2-propanol sont mélangés, entraînant la cristallisation de l'éthane-1,2-diaminium-bis[(2R)-2-bromo-3-phénylpropanoate], et
b) l'éthane-1,2-diaminium-bis[(2R)-2-bromo-3-phénylpropanoate] cristallisé est isolé.

6. Procédé selon la revendication 5, dans lequel le produit éthane-1,2-diaminium-bis[(2R)-2-bromo-3-phénylpropanoate] présente une pureté chimique plus élevée que la substance de départ acide (2R)-2-bromo-3-phénylpropionique.

7. Procédé selon la revendication 6, dans lequel la pureté chimique du produit éthane-1,2-diaminium-bis[(2R)-2-bromo-3-phénylpropanoate] est supérieure à 92,5 %.

8. Procédé selon l'une quelconque des revendications 6 à 7, dans lequel la pureté chimique du produit éthane-1,2-diaminium-bis[(2R)-2-bromo-3-phénylpropanoate] est d'au moins 98 %.

9. Procédé selon l'une quelconque des revendications 5 à 8, dans lequel l'éthane-1,2-diaminium-bis[(2R)-2-bromo-3-phénylpropanoate] cristallisé est isolé dans l'étape du procédé b), en le séparant par filtration ou en le filtrant à vide, en le lavant avec du 2-propanol et en le séchant.

10. Utilisation de l'éthane-1,2-diaminium-bis[(2R)-2-bromo-3-phénylpropanoate] selon l'une quelconque des revendications 1 à 4 pour la production d'inhibiteurs d'ACE/NEP.

11. Procédé de préparation d'un composé de formule (I) 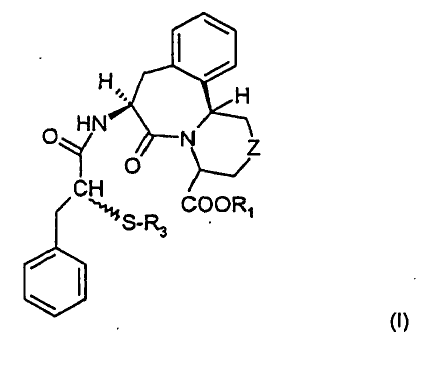 dans laquelle
R₁ représente un atome d'hydrogène, un groupe -CH₂O-C(O)C(CH₃)₃, un groupe alkyle en C₁-C₄ ou un groupe aryl-Y, dans lequel Y représente une liaison simple ou un groupe alkyle en C₁-C₄,
R₃ représente un atome d'hydrogène, un groupe acétyle, un groupe -CH₂O-C(O)C(CH₃)₃ ou un groupe benzoyle, et
Z représente un groupe (CH₂)ₙ, un groupe -O-, un atome de soufre, un groupe NR₆ ou un groupe N-C(O)R₇, où n représente un entier valant 0 ou 1, R₆ représente un atome d'hydrogène, un groupe alkyle en C₁-C₄ ou un groupe aryl-Y, et R₇ représente un groupe CF₃, un groupe alkyle en C₁-C₁₀ ou un groupe aryl-Y, où aryle représente un groupe phényle ou naphtyle, qui est non substitué ou substitué par de 1 à 3 substituants parmi le groupe constitué d'un groupe méthylènedioxy, d'un groupe hydroxy, d'un groupe alcoxy en C₁-C₄, d'un atome de fluore et d'un atome de chlore,
**caractérisé en ce que**
l'acide (2R)-2-bromo-3-phénylpropionique est d'abord libéré de l'éthane-1,2-diaminium-bis[(2R)-2-bromo-3-phénylpropanoate] sous forme d'intermédiaire ou dans une étape séparée, en ajoutant un acide, l'acide (2R)-2-bromo-3-phénylpropionique est ensuite mis à réagir avec un composé de formule (II) 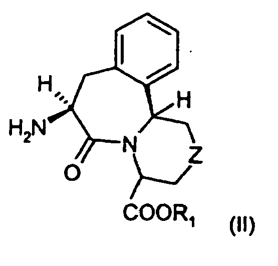 est mis à réagir avec un thioacétate pour donner lieu à un composé de formule (I), et le thioacétate est éventuellement mis à réagir pour donner lieu au thiol libre,
ou **caractérisé en ce que** l'acide (2R)-2-bromo-3-phénylpropionique est mis à réagir pour donner lieu à l'acide (S)-2-(acétylthio)-3-phénylpropionique, qui est ensuite mis à réagir avec un composé de formule (II) pour donner lieu à un composé de formule (I), et le thioacétate est éventuellement mis à réagir pour donner lieu au thiol libre.

12. Procédé selon la revendication 11, dans lequel le composé de formule (I) est décrit par un composé de formule (Ia) 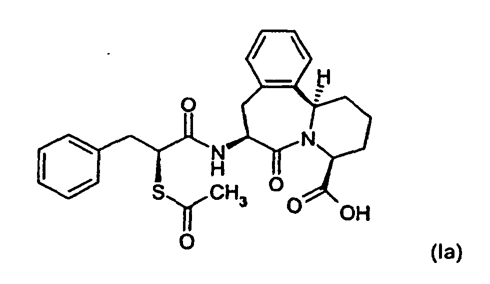 ou de formule (Ib)

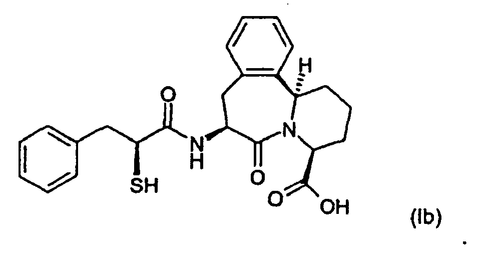

13. Procédé de préparation du composé de formule (III) 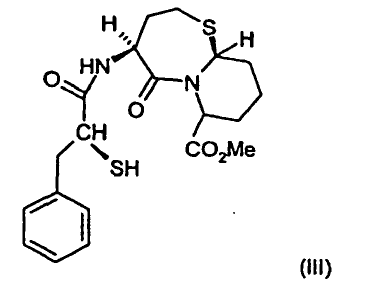 **caractérisé en ce que** l'acide (2R)-2-bromo-3-phénylpropionique est d'abord libéré de l'éthane-1,2-diaminium-bis[(2R)-2-bromo-3-phényl-propanoate], sous forme d'intermédiaire ou dans une étape séparée, en ajoutant un acide, l'acide (2R)-2-bromo-3-phénylpropionique est mis à réagir pour donner lieu à l'acide (S)-2-(acétylthio)-3-phénylpropionique, et dans une étape supplémentaire, l'acide (acétylthio)-3-phénylpropionique est mis à réagir avec un composé de formule (IV) 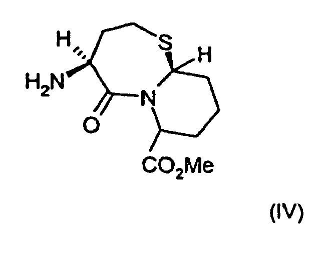 pour donner lieu au composé de formule (III).
